# DEMANDE DE BREVET EUROPEEN

(11) **EP 0 551 219 A1**
(43) Date de publication de la demande: **14.07.1993**
(21) Numéro de dépôt: 93400003.5
(22) Date de dépôt: 05.01.1993
(51) Int. Cl.: C07D 213/74, A61K 31/44

(54) **Nouveau dérivé de la pyridylsulfonylurée, ses procédés de préparation et les compositions pharmaceutiques le contenant**

(30) Priorité: 06.01.1992 FR 9200031
(71) Demandeur: ADIR ET COMPAGNIE, F-92415 Courbevoie Cédex (FR)
(72) Inventeur: Masereel, Bernard, B-6890 Libin (BE); Pirotte, Bernard, B-4680 Oupeye (BE); Schynts, Marc, B-4431 Loncin (BE); Delarge, Jacques, B-4140 Dolembreux (BE)

(57) **Abrégé**

L'invention concerne le dérivé de formule (I) 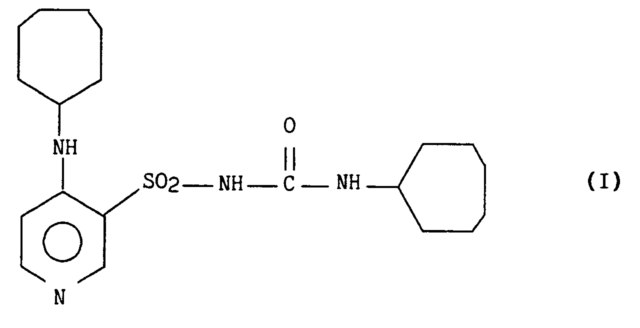
et ses sels d'addition à un acide ou à une base pharmaceutiquement acceptables.

Médicament.

## Description

L'invention concerne un nouveau composé dérivé de la pyridylsulfonylurée, ses procédés de préparation, et les compositions pharmaceutiques qui le contiennent.

Plus spécifiquement la présente invention concerne le composé de formule (I) : 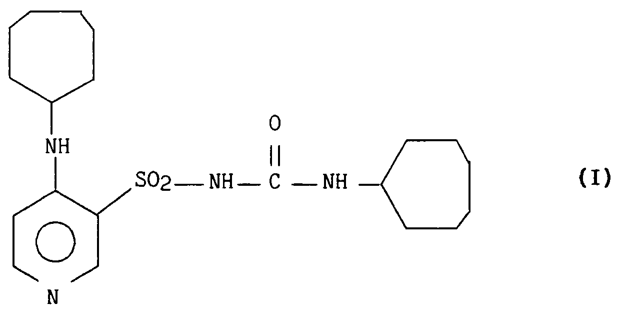
qui est la N-{[4-(cycloheptylamino)pyrid-3-yl]sulfonyl} N'-(cycloheptyl)urée, et ses sels d'addition à un acide ou à une base pharmaceutiquement acceptable.

On connait de l'art antérieur la demande de brevet européen EP 445 039 qui revendique des composés de formule générale (a) 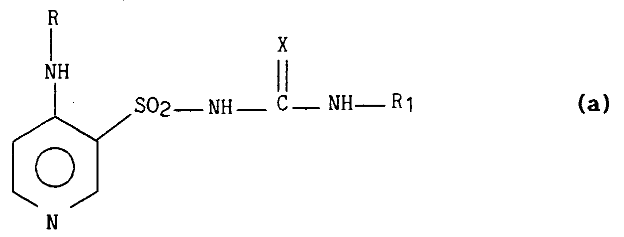
dans laquelle X peut représenter O ou S,
- R peut représenter un radical cycloalkyle contenant éventuellement un ou plusieurs hétéroatomes,
- et R₁ peut représenter un alkyle, ou un radical cycloalkyle contenant éventuellement un ou plusieurs hétéroatomes,

ces composés étant présentés comme antihypertenseurs et antioedèmateux. Les composés de la demande EP 445 039 se révèlent en effet être de puissants inhibiteurs du co-transport Na⁺/K⁺/2Cl⁻, l'inhibition de ce transporteur membranaire constituant le mécanisme d'action des diurétiques de l'anse. Ils montrent notamment une nette supériorité par rapport au Torasemide, un des diurétiques de l'anse les plus puissants connus actuellement. En outre, ils permettent, de façon originale, une remarquable épargne potassique.

Le composé de formule (I) est donc un cas particulier des composés de formule (a) où X est un atome d'oxygène, et où R et R₁ représentent chacun un radical cycloheptyle. Cependant, en aucun cas, ce composé n'apparait à l'homme de l'art, au vu de la demande EP 445 039, comme ayant un intérêt particulier puisque bien qu'englobé dans la formule générale de la revendication 1, il n'est ni décrit ni revendiqué spécifiquement.

Or la demanderesse a découvert que le composé de l'invention possède des activités pharmacologiques surprenantes.

La demanderesse a découvert que le composé de l'invention possède une activité antioedémateuse très puissante et surprenante par rapport au composé le plus proche de l'art antérieur.

La demanderesse a également découvert que le composé de formule (I) inhibe le canal Cl⁻ de façon remarquable et très supérieure (de plus de 50 %) par rapport au composé le plus proche de l'art antérieur.

En outre, la demanderesse a également découvert que le composé de l'invention associait à son activité antioedèmateuse une activité anti-hypoxique et anti-ischémique puissante. Cette activité était tout à fait inattendue puisque l'art antérieur, et notamment la demande EP 445 039, ne mentionne ni ne suggère une telle activité.

La toxicité du composé de l'invention a également été évalué chez le rat et la souris, il s'est avéré que le composé de l'invention est particulièrement bien toléré puisque à des doses supérieures à 3 200 mg/kg per os, il n'a été observé aucune mortalité.

La présente invention a également pour objet le procédé de préparation du composé de formule (I) caractérisé en ce que :
on fait réagir le [4-(cycloheptylamino)pyrid-3-yl]sulfonamide de formule (II) : 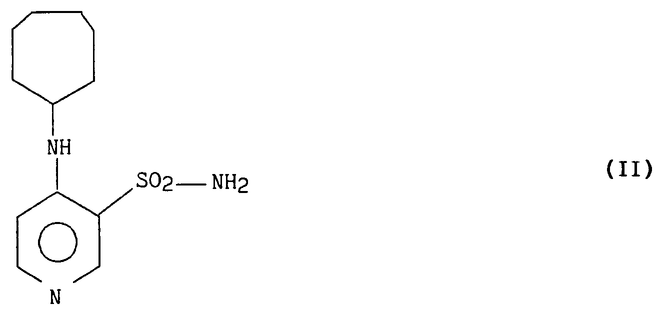
avec un composé de formule (III) :

Hal - CO - O - R₁ (III)

dans laquelle Hal représente un atome d'halogène et R₁ représente un alkyle contenant de 1 à 6 atomes de carbone, pour obtenir un composé de formule IV : 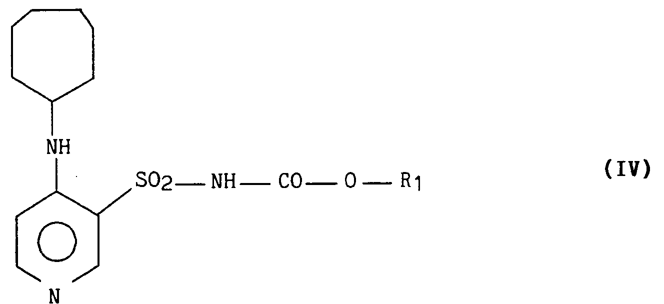
dans laquelle R₁ est tel que défini précédemment, qui est ensuite mis en réaction avec la cycloheptylamine pour conduire au composé de formule (I) qui est, si on le désire, purifié et transformé, le cas échéant, en son sel d'addition à un acide ou à une base pharmaceutiquement acceptable.

L'invention s'étend également à un second procédé de préparation du composé de formule (I) caractérisé en ce que :
on fait réagir, dans un solvant polaire, la [4-(cycloheptylamino)pyrid-3-yl]sulfonamide de formule II : 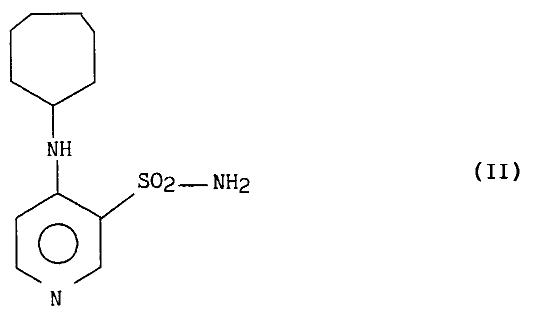
avec l'isocyanate de cycloheptyle de formule (V) 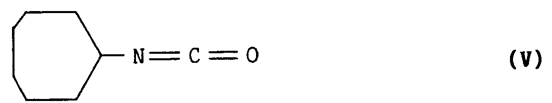
pour conduire au composé de formule (I) qui est, si on le désire, purifié et transformé, le cas échéant, en son sel d'addition à un acide ou à une base pharmaceutiquement acceptable.

Les matières premières utilisées dans les procédés précédemment décrits sont soit commerciales, soit aisément accessibles à l'homme de l'art selon des procédés décrits dans la littérature, notamment dans la demande EP 445 039.

Parmi les acides pharmaceutiquement acceptables que l'on peut utiliser pour former un sel d'addition avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases pharmaceutiquement acceptables que l'on peut utiliser pour former un sel d'addition avec les composés de l'invention, on peut citer, à titre d'exemples et de façon non limitative, les hydroxydes de sodium, de potassium, de calcium, ou d'aluminium, la triéthylamine, la benzylamine, la diéthanolamine, la tert-butylamine, la dicyclohexylamine, et l'arginine.

La demanderesse a découvert que le composé de l'invention possède une activité pharmacologique très intéressante et surprenante au vu du composé le plus proche de l'art antérieur.

En effet, le composé de l'invention possède une activité antioedémateuse très puissante (Exemple 3 de la présente demande : Etude de l'activité antioedémateuse chez le lapin), et surprenante par rapport au composé de l'exemple 8 de la demande EP 445 039 (la N-{[4-(cyclooctylamino)pyrid-3-yl]sulfonyl} N'-(cycloheptyl)urée) qui est le composé de l'art antérieur le plus proche structuralement. Le composé de cet exemple 8 ne diffère en effet que par la présence d'un chainon carboné supplémentaire dans le groupement cycloalkyle en position 4 du noyau pyridique.

En outre, l'activité inhibitrice du composé de l'invention sur le canal Cl⁻(un des acteurs intervenant dans la prise d'eau cellulaire et donc dans l'oedème) est aussi très supérieure à celle du composé de l'exemple 8 de la demande EP 445 039 (la N-{[4-(cyclooctylamino)pyrid-3-yl]sulfonyl} N'-(cycloheptyl)urée), qui est le composé de l'art antérieur le plus proche structuralement et qui est également le composé apparaissant le plus actif dans l'inhibition du canal Cl⁻ (exemple 19 de la demande EP 445 039).

D'autre part, la demanderesse a découvert que le composé de l'invention possédait également une activité anti-ischémique et anti-hypoxique remarquable et très puissante.

Le composé de l'invention permet en effet d'obtenir, tant in vitro qu'in vivo, une protection étonnante contre l'hypoxie (test d'hypoxie sur des astrocytes en culture et test d'hypoxie normobare sur souris, décrits dans notre étude pharmacologique : exemples 5 et 6 de la présente demande) et contre l'ischémie (test de l'ischémie provoquée chez la gerbille : exemple 7 de la présente demande).

Une telle activité anti-hypoxique et anti-ischémique du composé de l'invention est inattendue puisque la demande EP 445 039 ne mentionne ni ne suggère en aucun cas de telles propriétés. Ceci est confirmé par l'étude pharmacologique (exemples 5, 6 et 7 de la présente demande) du composé de l'exemple 8 de la demande EP 445 039 (la N-{[4-(cyclooctylamino)pyrid-3-yl]sulfonyl} N'-(cycloheptyl)urée) qui montre que ce composé ne possède aucune activité anti-hypoxique, et ce même à des doses plus importantes, ce qui renforce l'aspect surprenant des propriétés protectrices du composé de l'invention.

Les remarquables propriétés pharmacologiques du composé de la présente invention le rendent notamment précieux dans la prévention et le traitement des troubles ischémiques et hypoxiques et des oedèmes périphériques et centraux. Les composés de l'invention sont donc utiles dans le traitement et la prévention de l'ischémie cérébrale, de l'hypoxie vasculaire cérébrale, de l'anoxie vasculaire cérébrale, des traumatismes crâniens, des encéphalopathies, des maladies neuro-végétatives, des convulsions post-ischémiques, et des troubles de la sénéscence, ainsi que pour le traitement et la prévention de l'ischémie de type périphérique, et en cardiologie de l'ischémie myocardique et de l'ischémie coronarienne ainsi que de leurs différentes expressions cliniques : angine de poitrine, infarctus du myocarde, troubles du rythme, spasme vasculaire, insuffisance cardiaque, ainsi qu'en ophtalmologie et en oto-rhino-laryngologie lors d'atteintes vasculaires chorio-rétiniennes, de vertiges d'origine vasculaire, de vertiges de Ménière ou d'acouphènes.

L'invention s'étend aussi aux compositions pharmaceutiques renfermant comme principe actif le composé de formule (I) ou un des ses sels d'addition, à un acide ou à une base pharmaceutiquement acceptable, en combinaison avec un ou plusieurs excipients pharmaceutiquement acceptables

Parmi les compositions selon l'invention, on pourra citer, à titre d'exemples et de façon non limitative, celles qui conviennent pour l'administration orale, parentérale, oculaire, per ou trans-cutanée, nasale, rectale, perlinguale, ou respiratoire, et notamment les préparations injectables, les aérosols, les gouttes oculaires ou nasales, les comprimés, les comprimés sublinguaux, les gélules, les capsules, les tablettes, les glossettes, les suppositoires, les crèmes, les pommades, les gels.

Les préparations ainsi obtenues se présentent généralement sous forme dosée et peuvent contenir selon les affections traitées, l'âge, et le sexe du malade, de 0,01 à 100 mg de principe actif (préférentiellement de 0,01 à 10 mg, par exemple de 0,01 à 0,1 mg) de une à trois fois par jour.

Les exemples suivants illustrent l'invention et ne la limitent en aucune manière.

### EXEMPLE 1 :

### N-{[4-(cycloheptylamino)pyrid-3-yl]sulfonyl} N'-(cycloheptyl)urée

### Stade A : N-{[4-(cycloheptylamino)pyrid-3-yl]sulfonyl} N'-(éthoxycarbonyl)amine

On met en suspension 3 g de [4-(cycloheptylamino)pyrid-3-yl] sulfonamide dans 20 cm³ de tétrahydrofurane anhydre et on ajoute un équivalent de triéthylamine. On porte le mélange à 50°C. On ajoute ensuite rapidement 10 cm³ de chloroformiate d'éthyle. Il se produit un violent reflux. On maintient alors le reflux et l'agitation durant 20 mn.

On filtre le mélange réactionnel et on évapore le filtrat sous dépression. Le résidu est ensuite mis en solution dans un mélange eau/acétone (90/10) contenant du NaHCO₃.

Après filtration, le filtrat est ensuite amené à pH 6,5. La N-{[4-(cycloheptylamino)pyrid-3-yl]sulfonyl} N'-(éthoxycarbonyl)amine précipitée est essorée et séchée sous vide.
Rendement : 55 %

### Stade B : N-{[4-(cycloheptylamino)pyrid-3-yl]sulfonyl} N'-(cycloheptyl)urée

On dissout 3 g du composé obtenu au stade A dans 40 cm³ de toluène anhydre et 2 cm³ de cycloheptylamine. On ajoute également 3 g de tamis moléculaire (0,4 nm).

On porte la solution à reflux et on suit l'évolution de la réaction par chromatographie sur couche mince.

Au terme de la réaction, on évapore sous vide le toluène et on reprend le résidu par un mélange eau/NaOH (90/10).

On extrait l'excès d'amine dans la solution alcaline par de l'éther puis la phase aqueuse est clarifiée au charbon, filtrée, et amenée jusqu'à un pH de 6,5 avec de l'acide chlorhydrique.

La N-{[4-(cycloheptylamino)pyrid-3-yl]sulfonyl} N'-(cycloheptyl)urée ainsi obtenue est essorée, puis séchée, et peut être éventuellement recristallisée dans une solution hydroalcoolique.
Rendement : 64 %
Point de fusion : 172-174°C

### Microanalyse :

| **%** | **C** | **H** | **N** | **S** |
|---|---|---|---|---|
| calculé | 58,80 | 7,89 | 13,71 | 7,85 |
| trouvé | 58,75 | 7,83 | 13,92 | 7,66 |

### EXEMPLE 2 :

### N-{[4-(cycloheptylamino)pyrid-3-yl]sulfonyl} N'-(cycloheptyl)urée, 2ème procédé

On ajoute 0,01 mole de NaOH en solution dans un minimum d'eau à une solution de 2,4 g de (4-(cycloheptylamino)pyrid-3-yl)sulfonamide dans 80 cm³ d'un mélange eau-acétone (1/1). On agite avec un barreau magnétique et on ajoute 2,1 g de cycloheptylisocyanate. On maintient sous agitation en suivant l'évolution de la réaction par chromatographie sur couche mince. On évapore sous vide et on reprend le résidu par 100 cm³ de NaOH 0,2 N. Après filtration, la solution est amenée à un pH de 6,5, et le précipité résultant est essoré puis séché. La N-{[4-(cycloheptylamino)pyrid-3-yl]sulfonyl} N'-(cycloheptyl)urée ainsi obtenue peut être éventuellement recristallisée dans une solution hydroalcoolique.
Rendement : 68 %
Point de fusion : 172-174°C

### EXEMPLE 3 :

### Etude de l'activité anti-oedémateuse in vivo chez le lapin

L'oedème est provoqué in vivo en soumettant les animaux à des épisodes hypoxiques de 30 mn par occlusion bilatérale des artères carotides communes. L'analyse comparative est ensuite réalisée au niveau des lobes frontaux entre les animaux témoins et les animaux traités par les composés à tester.

### Protocole :

### Etude in vivo

Les expériences sont réalisées sur des lapins adultes (Fauves de Bourgogne, de 2 - 2,5 kg). Sept animaux sont utilisés par situation expérimentale. Le gonflement astrocytaire est induit en soumettant le cerveau des animaux à des épisodes hypoxiques de 30 mn par occlusion bilatérale des artères carotides communes. Les études ultrastructurales sont effectuées sur échantillons prélevés au niveau des lobes frontaux de lapins témoins, hypoxiés, et traités-hypoxiés.

Les animaux, préanesthésiés à la chlorpromazine (65 mg/kg) et anesthésiés au pentobarbital sodique (20 mg/kg) reçoivent la substance à tester, par injection dans la veine marginale de l'oreille, 10 mn avant le début de l'hypoxie. La dose initiale de la substance à tester est de 10 mg/kg.

A l'issue de l'épisode hypoxique, les animaux sont sacrifiés par perfusion intracardiaque de la solution fixatrice (glutaraldéhyde à 2,5 % en tampon phosphate 0,1 M, pH 7,4).

Les échantillons prélevés poursuivent leur fixation dans le même fixateur durant 1 heure, sont lavés en tampon phosphate 0,1 M additionné de saccharose 0,18 M, traités par du tétroxide d'osmium à 1 % en tampon phosphate, puis déshydratés, inclus et coupés sur ultratome à l'aide d'un couteau de diamant ; après quoi les coupes, montées sur grilles, sont observées sur un microscope électronique.

### Résultats :

L'analyse ultrastructurale des coupes sur un microscope électronique établit la présence chez les animaux hypoxiés d'un oedème cérébral caractéristique (gonflement des astrocytes, altération des noyaux et des mitochondries).

Le composé de l'invention s'avère posséder une puissante activité antioedémateuse puisque les coupes cérébrales issues des animaux prétraités par 10 mg/kg du composé de l'invention ne laisent apparaître aucun oedème.

Par contre, à cette même dose de 10 mg/kg, le composé de l'exemple 8 de la demande EP 445 039, composé le plus proche structuralement de l'art antérieur, procure seulement une faible protection anti-oedémateuse.

### EXEMPLE 4 :

### Etude de l'activité inhibitrice du canal Cl⁻

Cette étude permet de déterminer la concentration des composés inhibant à 50 % (IC₅₀) le canal Cl⁻. Ce test a été réalisé sur le canal Cl⁻ présent sur la membrane basolatérale de la branche ascendante de l'anse de Henlé du néphron de lapin perfusé, et l'amplitude de l'inhibition est déterminée par électrophysiologie.

Le composé de l'invention a été comparé au Torasémide qui est un des plus puissants diurétiques connus actuellement agissant au niveau de la branche ascendante de l'anse de Henlé.

Il est également comparé au composé de l'exemple 8 de la demande EP 445 039 (la N-{[4-(cyclooctylamino)pyrid-3-yl]sulfonyl} N'-cycloheptylurée).

Les résultats montrent de façon surprenante la supériorité du composé de l'invention dans l'inhibition du canal Cl⁻ puisque son IC₅₀, c'est-à-dire la concentration nécessaire pour 50 % d'inhibition, est inférieure de 80 % à celle du Torasémide et inférieure de plus de 50 % à celle de l'exemple 8 de la demande EP 445 039, qui est le composé le plus proche de l'art antérieur.

### EXEMPLE 5 :

### Etude de l'activité anti-hypoxique in vitro

Les astrocytes en culture constituent un modèle de choix pour la recherche d'une activité cytoprotectrice face à une situation d'hypoxie.

En effet, la première réaction cellulaire visible à toute agression cérébrale est une atteinte de l'intégrité astrocytaire, alors même que les neurones, les oligodendrocytes, et les cellules endothéliales ont encore un profil morphologique normal.

En outre, il a été montré que l'astrocyte avait un rôle majeur au niveau cérébral, notamment dans l'élaboration des acides aminés neurotransmetteurs et dans la préservation de l'équilibre ionique extracellulaire.

La demanderesse a donc testé l'effet des composés de l'invention sur la protection cellulaire d'astrocytes en culture placés en situation d'hypoxie en analysant un marqueur enzymatique (la lactate deshydrogénase ou LDH) qui permet de mesurer la lyse cellulaire des astrocytes.

### Méthodologie :

Les astrocytes de rats en culture primaire sont préparés à partir de cortex provenant de cerveaux de rats nouveau-nés. Le traitement hypoxique consiste en une exposition des cellules, en atmosphère humide, à un mélange gazeux constitué de 95 % N₂ et 5 % CO₂, à 37°C pendant 15 heures.

Les composés à tester sont ajoutés au milieu de culture 12 heures avant l'hypoxie. Un second ajout est réalisé à la fin de la période hypoxique. Deux heures après la fin de l'hypoxie, on mesure l'activité lactate deshydrogénase extracellulaire par dosage spectrophotométrique à 340 nm sur le milieu de culture.

### Résultats :

Le composé de l'invention présente une activité anti-hypoxique exceptionnelle puisqu'il permet d'éviter la lyse cellulaire, normalement causée par l'hypoxie et mesurée dans l'expérience contrôle sans produit.

Ce résultat est surprenant puisque ni le furosémide ni l'un des composés le plus proche de l'art antérieur (l'exemple 8 de la demande EP 445 039, qui est le N-{[4-(cyclooctylamino)pyrid-3-yl]sulfonyl} N'-(cycloheptyl)urée n'ont d'action sur cette lyse cellulaire.

A titre d'exemple, la comparaison entre le composé de l'invention et le furosémide après une période de 18 h d'hypoxie montre que, à une concentration de 10 µM, le composé de l'invention permet une protection astrocytaire proche de 90 % alors que le furosémide, à cette même concentration, n'a aucune activité protectrice.

Ces résultats montrent l'intérêt du composé de l'invention dans le traitement des traumatismes hypoxiques cérébraux et des pathologies associées.

### EXEMPLE 6

### Etude de l'activité anti-hypoxique in vivo

Des animaux (souris) sont placés dans une atmosphère pauvre en oxygène, ce qui provoque l'apparition de suffocations.

Les composés possédant des propriétés anti-hypoxiques provoquent un retard dans l'apparition de ces suffocations.

La demanderesse a présentement testé le composé de l'invention dans ce test.

### Méthodologie :

Les souris mâles (Swiss CD1) pesant 25-30 g sont stabulées durant 1 semaine avant toute expérience dans les conditions usuelles d'animalerie (20-22°C, 55 % d'humidité, cycle lumière/obscurité 12/12, nourriture industrielle et eau à volonté).

Les souris sont placées dans une boite (7 × 5 × 5 cm) dans laquelle on crée une atmosphère pauvre en oxygène par le passage d'un air à 96 % N₂ et 4 % O₂.

Le délai d'apparition des premières suffocations est mesuré. Les souris reçoivent une dose des composés à tester par voie intrapéritonéale.

### Résultats :

Le composé de l'invention présente une activité anti-hypoxique significative dès la dose de 0,1 mg/kg alors que le composé le plus proche de l'art antérieur (l'exemple 8 de la demande EP 445 039, la N-{[4-(cyclooctylamino)pyrid-3-yl]sulfonamide} N'-(cycloheptyl)urée ne possède aucune activité antihypoxique même à une dose de 20 mg/kg, c'est-à-dire une dose 200 fois supérieure.

### EXEMPLE 7 :

### Recherche d'une activité anti-ischémique in vivo.

Certaines gerbilles (40 à 60 % des cas), dites sensibles, présentent une anomalie du cercle de Willis (Levine et al. Exp. Neurol. 1966 ; 16 : 255-262).

Grâce à cette anomalie, l'occlusion d'une carotide chez la gerbille permet de reproduire, contrairement aux autres espèces animales, la pathologie de l'ischémie humaine.

La demanderesse a donc testé l'influence du composé de l'invention sur la survie des gerbilles ayant subi une ischémie cérébrale par ligature de la carotide gauche.

### Méthodologie :

On anesthésie les gerbilles "sensibles" au Kétalar® à la dose de 60 mg/kg par voie intrapéritonéale et 30 minutes avant la ligature de la carotide gauche, on administre, par la voie orale, différentes concentrations du composé de l'invention en solution en présence de gomme arabique à 10 % et sous un volume de 0,1 cm³.

On analyse le comportement des animaux à différents temps.

### Résultats :

Le composé de l'invention possède une activité protectrice anti-ischémique très importante.

En effet, au temps 96 h, alors que tous les animaux sont morts dans l'expérience contrôle, le composé de l'invention, administré per os, dès la dose de 0,1 mg/kg, permet d'obtenir un fort pourcentage de survie (83 % à 0,1 mg/kg, activité très supérieure à celle du composé le plus proche de l'art antérieur (l'exemple 8 de la demande EP 445 039)).

En outre, l'étude des animaux traités montre que le composé de l'invention permet, jusqu'au temps 96 h, une protection comportementale significative. Le composé de l'invention possède notamment une forte activité inhibitrice des convulsions post-ischémiques.

### EXEMPLE 8 :

### Composition pharmaceutique

Comprimés contenant 1 mg de N-{[4-(cycloheptylamino)pyrid-3-yl]sulfonyl} N'-(cycloheptyl)urée
Formule pour 1 000 comprimés
N-{[4-(cycloheptylamino)pyrid-3-yl]sulfonyl} N'-(cycloheptyl)urée 1 gramme
- lactose 15 grammes
- amidon de blé 50 grammes
- silice colloïdale 0,2 gramme
- stéarate de magnésium 0,1 gramme

## Revendications

**1/** Composé de formule (I) 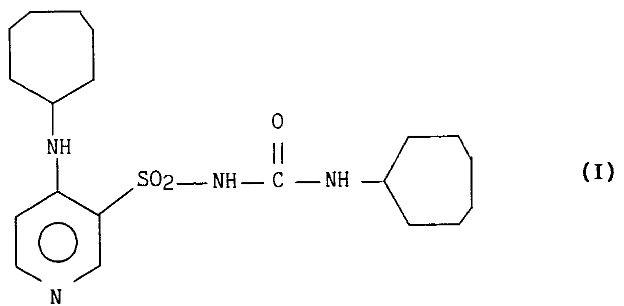 qui est la N-{[4-(cycloheptylamino)pyrid-3-yl]sulfonyl} N'-(cycloheptyl)urée ainsi que ses sels d'addition à un acide ou à une base pharmaceutiquement acceptable.

**2/** Procédé de préparation du composé de formule (I) selon la revendication 1 caractérisé en ce que :
on fait réagir la [4-(cycloheptylamino)pyrid-3-yl]sulfonamide de formule (II) : 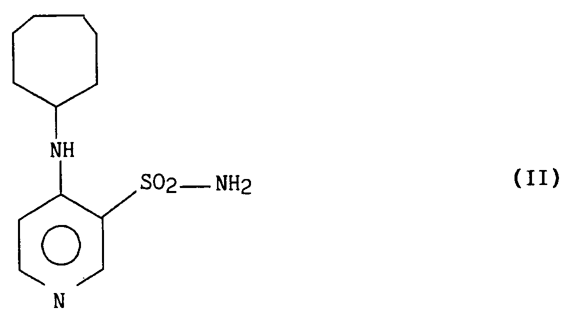 avec un composé de formule (III) :
Hal - CO - O - R₁ (III)
dans laquelle Hal représente un atome d'halogène et R₁ représente un alkyle contenant de 1 à 6 atomes de carbone, pour obtenir un composé de formule (IV) : 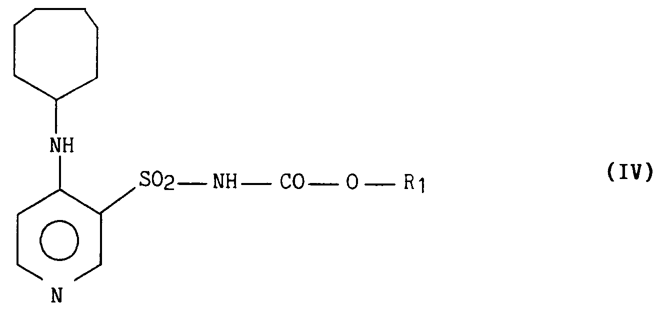 dans laquelle R₁ est tel que défini précédemment, qui est ensuite mis en réaction avec la cycloheptylamine pour conduire au composé de formule (I) selon la revendication 1, composé de formule (I) qui est, si on le désire, purifié et transformé, le cas échéant, en son sel d'addition à un acide ou à une base pharmaceutiquement acceptable.

**3/** Procédé de préparation du composé de formule (I) selon la revendication 1 caractérisé en ce que :
on fait réagir, dans un solvant polaire, la [4-(cycloheptylamino)pyrid-3-yl]sulfonamide de formule (II) : 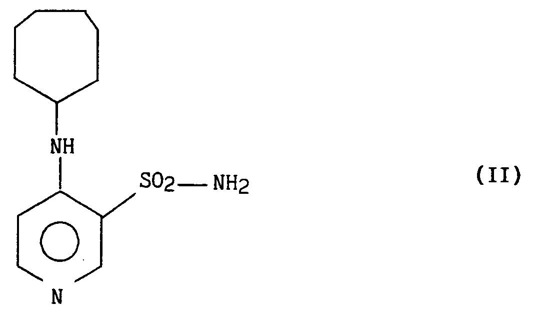 avec l'isocyanate de cycloheptyle de formule (V) 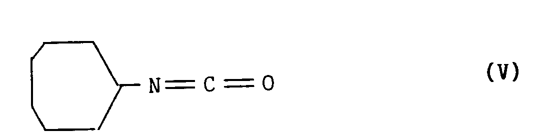 pour conduire au composé de formule (I) selon la revendication 1, composé de formule (I) qui est, si on le désire, purifié et transformé, le cas échéant, en son sel d'addition à un acide ou à une base pharmaceutiquement acceptable.

**4/** Composition pharmaceutique renfermant comme principe actif le composé de formule (I) selon la revendication 1 en combinaison avec un ou plusieurs excipients ou véhicules pharmaceutiquement acceptables.

**5/** Composition pharmaceutique selon la revendication 4 utilisable dans le traitement et la prévention des troubles ischémiques et hypoxiques et des oedèmes périphériques ou centraux.

**6/** Composition pharmaceutique selon la revendication 4 utilisable dans le traitement et la prévention des troubles ischémiques et hypoxiques.

**7/** Composition pharmaceutique selon la revendication 4 utilisable dans le traitement et la prévention de l'ischémie cérébrale, de l'hypoxie vasculaire cérébrale, de l'anoxie vasculaire cérébrale, des traumatismes crâniens, des encéphalopathies, des maladies neuro-végétatives, des convulsions post-ischémiques, et des troubles de la sénescence, ainsi que pour le traitement et la prévention de l'ischémie de type périphérique, et en cardiologie de l'ischémie myocardique et de l'ischémie coronarienne ainsi que de leurs différentes expressions cliniques : angine de poitrine, infarctus du myocarde, troubles du rythme, spasme vasculaire, insuffisance cardiaque, ainsi qu'en ophtalmologie et en oto-rhino-laryngologie lors d'atteintes vasculaires chorio-rétiniennes, de vertiges d'origine vasculaire, de vertiges de Menière ou d'acouphènes.
